# EUROPEAN PATENT SPECIFICATION

(11) **EP 4 267 090 B1**
(45) Date of publication and mention of the grant of the patent: **05.08.2026**
(21) Application number: 20853518.7
(22) Date of filing: 23.12.2020
(51) Int. Cl.: A61K 8/34, A61K 8/9789, A61Q 19/00, A61Q 19/08

(54) **COSMETIC COMPOSITION COMPRISING AN AQUEOUS OR AN HYDROALCOHOLIC EXTRACT OF PEONY**
KOSMETISCHE ZUSAMMENSETZUNG ENTHALTEND EIN WÄSSRIGES ODER HYDROALKOHOLISCHES EXTRAKT AUS PFINGSTROSEN
COMPOSITION COSMÉTIQUE COMPRENANT UN EXTRAIT AQUEUX OU HYDROALCOOLIQUE DE PIVOINE

(43) Date of publication of application: 01.11.2023
(73) Proprietor: L V M H RECHERCHE, 45800 St. Jean de Braye (FR)
(72) Inventor: CHOISY, Patrick, 45800 Saint Jean de Braye (FR); GOURGUILLON, Lorène, 45800 Saint Jean de Braye (FR); NIZARD, Carine, 45800 Saint Jean de Braye (FR); LEBLANC, Emmanuelle, 45800 Saint Jean de Braye (FR)
(74) Representative: Regimbeau
(86) International application number: PCT/IB2020/001128
(87) International publication number: WO 2022/136897

(56) References cited:
- EP-A2- 2 246 037
- CN-A- 102 209 527
- CN-A- 108 379 168
- KR-A- 20170 136 378
- US-A1- 2015 342 854
- CHONGHUI LI ET AL: "Flavonoid Composition and Antioxidant Activity of Tree Peony (Paeonia Section Moutan ) Yellow Flowers", JOURNAL OF AGRICULTURAL AND FOOD CHEMISTRY, vol. 57, no. 18, 23 September 2009 (2009-09-23), pages 8496 - 8503, XP055013735, ISSN: 0021-8561, DOI: 10.1021/jf902103b
- PAN YANG ET AL: "Chemical and biological comparison of different parts of Paeonia suffruticosa (Mudan) based on LCMS-IT-TOF and multi-evaluation in vitro", INDUSTRIAL CROPS AND PRODUCTS, ELSEVIER, NL, vol. 144, 25 December 2019 (2019-12-25), XP085983895, ISSN: 0926-6690, [retrieved on 20191225], DOI: 10.1016/J.INDCROP.2019.112028

## Description

### FIELD OF THE INVENTION

The present invention relates to a cosmetic composition comprising an aqueous or hydroalcoholic extract of *Paeonia suffruticosa* Yao Huang, and the use thereof for anti-aging, in particular to promote and/or improve the barrier function, densify the extracellular matrix and/or reduce its degradation, improve the elasticity and/or firmness of the skin, improve the radiance and/or homogeneity of the skin, improve the collagen synthesis in the skin.

### PRIOR ART

The skin is the primary barrier of the human body. It protects the organs from changes in temperature and humidity and from damage from the external environment, such as UV radiation or pollutants. However, excessive chemical and physical stimulation are harmful to the normal functions of the skin and cause aging. This extrinsic aging leads to alterations such as deep wrinkles and the formation of skin that has lost its firmness, its suppleness and its elasticity. These transformations are essentially due to histological changes, such as an excessive modification of the elastic tissue and a quantitative and qualitative degeneration of the collagen fibers.

Concomitantly, intrinsic aging, "normal" or chronobiological aging, is the consequence of planned senescence in which endogenous factors play a part. This intrinsic aging notably causes the renewal of skin cells, the keratinocytes, to slow down, which is essentially translated by the appearance of changes such as the reduction of subcutaneous adipose tissue and the appearance of fine lines or wrinkles, and by histopathological changes such as an increase in the number and thickness of elastic fibers, a loss of vertical fibers of the elastic tissue membrane, and the presence of large irregular fibroblasts in the elastic tissue cells.

The present invention applies to intrinsic and extrinsic aging.

The search for new molecules or active ingredients usable in cosmetics is a necessity in order to develop efficient products to give a younger appearance to the skin, to attenuate wrinkles, to smooth the skin and to brighten the skin tone.

For delaying and reducing the effect of skin aging, the plant extracts are frequently used and preferred for their natural aspect and for their obtention relatively easy. However, it is still preferable to provide new plant extracts having an improved anti-aging effect. US 2015/342854 describes an anti-aging composition with vitamin C and further describes *Paeonia suffructicosa* as a moisturizing, anti-inflammatory and blood circulation promoting agent; CN 108 379 168 describes anti-aging compositions with aqueous or alcoholic extracts of peony, although not of *Paeonia suffructicosa.*

Surprisingly, the Applicant has shown that extracts from particular species of *Paeonia* and particularly extracts from the specie called *Paeonia suffruticosa* Yao Huang (cultivated in China) have an activity of primary importance for acting effectively against the skin aging. By means of an evaluation of fibroblasts cultures, he has demonstrated the properties of hydroalcoholic extracts of *Paeonia suffruticosa* Yao Huang as an active agent that can fight the effects or the appearance of signs of intrinsic and/or extrinsic skin aging, in particular by decreasing the synthesis of metalloproteinase and ROS formation and by increasing the synthesis of collagen, particularly collagen I.

### SUMMARY OF THE INVENTION

A first subject matter of the invention relates to a cosmetic composition for topical application to the skin comprising, in a physiologically acceptable medium, at least one effective amount of at least one aqueous or hydroalcoholic extract of *Paeonia suffruticosa* Yao Huang, preferably an hydroalcoholic extract of *Paeonia suffruticosa* Yao Huang.

Another subject matter of the invention relates to a cosmetic process for caring the keratinic materials of a subject in need thereof, in particular skin and/or lips, comprising the application to the keratinic materials, in particular skin and/or lips of the cosmetic composition of the invention. Particularly, the cosmetic composition is applied onto keratinic materials of a subject in need thereof, for anti-aging, to promote and/or improve the barrier function, densify the extracellular matrix and/or reduce its degradation, improve the elasticity and/or firmness of the skin, improve the radiance and/or homogeneity of the skin, improve the collagen synthesis in the skin and/or prevent and/or reduce the degradation of collagen, reduce the production of metalloproteases, and/or reduce the formation of wrinkles and/or fine lines.

The invention also relates to a non-therapeutic cosmetic use of at least one effective amount of at least one aqueous or hydroalcoholic extract of *Paeonia suffruticosa* Yao Huang, in particular an hydroalcoholic extract of *Paeonia suffruticosa* Yao Huang, as an agent for anti-aging, intended to promote and/or improve the barrier function, densify the extracellular matrix and/or reduce its degradation, improve the elasticity and/or firmness of the skin, improve the radiance and/or homogeneity of the skin, improve the collagen synthesis in the skin and/or prevent and/or reduce the degradation of collagen, reduce the production of metalloproteases, and/or reduce the formation of wrinkles and/or fine lines.

A further subject matter of the invention relates to an aqueous or hydroalcoholic extract of *Paeonia suffruticosa* Yao Huang obtained from the whole plant, or preferably from the leaves, from the flowers, from the roots or from the seed pods, more preferably from the leaves or from the flowers and using a cosmetically acceptable polar solvent selected from C1-C5 mono-alcohol and water, in particular a mixture of ethanol and water, more preferably in a mass ratio between 40 ethanol/60 water and 60 ethanol/40 water.

The present invention also relates to an extraction process for obtaining the hydroalcoholic extract of *Paeonia suffruticosa* Yao Huang, said extraction process comprising the following steps:
a) harvesting whole fresh plants of *Paeonia suffruticosa* Yao Huang, optionally stored at -20°C,
b) separating the flowers, the leaves, the roots and the seed pods;
c) freeze drying the plant parts for 24 hours at - 45°C
d) macerating 5 to 20%, in particular 10% of plant material in a solvent comprising 30% to 50% water, 50% to 70% ethanol, particularly at temperature comprised between 40°C and 60°C,
e) extraction optionally under stirring,
f) liquid/solid separation,
g) optional addition of preservatives,
h) filtration, especially to 0.22 µm,
i) optionally packaging, under nitrogen if necessary,
j) optionally storage at +4°C.

### DETAILED DESCRIPTION OF THE INVENTION

As indicated above, the invention relates to a cosmetic composition for topical application to the skin comprising, in a physiologically acceptable medium, at least one effective amount of at least one aqueous or hydroalcoholic extract of *Paeonia suffruticosa* Yao Huang, preferably an hydroalcoholic extract of *Paeonia suffruticosa* Yao Huang.

The aqueous or the hydroalcoholic extract of *Paeonia suffruticosa* Yao Huang is present in the cosmetic composition of the invention in an effective amount to obtain the desired effect.

Particularly, the aqueous or the hydroalcoholic extract of *Paeonia suffruticosa* Yao Huang is present in the composition in a content ranging from 0.01% to 95%, in particular from 0.01% to 20%, preferably from 0.05% to 10% and more preferably from 0,05% to 5% by weight of raw material based on the total weight of said composition.

### Plant material

According to the invention, the aqueous or the hydroalcoholic extract is obtained *Paeonia suffruticosa* Yao Huang.

*Paeonia suffruticosa Yao Huang is a particular cultivar of the tree Paeonia (Paeonia suffruticosa*) frequently cultivated as ornamental flower in China.

According to one embodiment of the invention, the aqueous or hydroalcoholic extract of *Paeonia suffruticosa* Yao Huang is an extract obtained from the whole plant. Particularly, the extract is obtained from the leaves, from the flowers, from the roots or from the seed pods, more preferably from the leaves, from the flowers or from the roots.

The plant of *Paeonia suffruticosa* Yao Huang can be harvested and directly frozen; these will be referred to as 'wet' or 'fresh' frozen plant or plant's parts (flowers, leaves, seed pods and roots). According to another embodiment, the plant of *Paeonia suffruticosa* Yao Huang can be harvested and dried (freeze-dried) before being frozen; these will be referred to as frozen 'freeze-dried' plant or plant's parts (flowers, leaves, seed pods and roots).

According to a particular and preferred embodiment, the whole plant of *Paeonia suffruticosa* Yao Huang or their parts can be freeze-dried using conventional methods of freezing-draying.

In a particular and preferred embodiment, the extract of *Paeonia suffruticosa* Yao Huang is an extract from flowers, leaves or roots, preferably flowers or leaves.

### Aqueous and hydroalcoholic extract of Paeonia Yao Huang

### ➢ Aqueous extract

The terms aqueous extract or polar extract or hydrophilic extract of *Paeonia suffruticosa* Yao Huang are used interchangeably in the description. Aqueous extract of *Paeonia suffruticosa* Yao Huang is obtained using a cosmetically acceptable polar solvent.

The term 'aqueous extract of *Paeonia* Yao Huang' according to the invention means in particular that the polar (hydrophilic) compounds of the *Paeonia suffruticosa* Yao Huang have been solubilized and/or extracted in a polar solvent.

Advantageously, the extract of the invention is obtained by 'Eco extraction' based on the discovery and design of extraction processes to reduce energy consumption, but also the use of alternative solvents and renewable plant resources, while ensuring a safe and quality product/extract. Eco-extraction is based on 6 main principles (Farid Chemat, Dunod, 2010, Eco-Extraction du végétal):
- Promote innovation through varietal selection and the use of renewable plant resources,
- Prefer alternative solvents known as green solvents and mainly those from agro-resources,
- Reduce energy consumption through the assistance of innovative technologies and promote energy recovery,
- Encourage the creation of co-products instead of waste to integrate the organic or agro-refinery route,
- Reduce unit operations through technological innovation and promote safe, robust and controlled processes, and
- Prefer a product that is undenatured, biodegradable, contaminant-free and above all, has "eco-Extract" values. It is therefore advantageous to use water as a polar green solvent.

Prior to the extraction step itself, the whole plant of its parts (flowers, leaves, roots and seed pods) may have been dried and/or ground. According to a particular embodiment, the plant or plant's part are dried at room temperature.

Moreover, the ground before extraction, is performed for example by means of a mortar, a cryo-grinder, a mixer, a traditional mill or a centrifuge according to the methods known to the skilled person.

The aqueous or hydroalcoholic extract of *Paeonia suffruticosa* Yao Huang is obtained from the whole plant, or preferably from the leaves, from the flowers, from the roots or from the seed pods, more preferably from the leaves or from the flowers using a cosmetically acceptable polar solvent selected from example C1-C5 mono-alcohol and water. Particularly, a mixture of ethanol and water, more preferably in a mass ratio between 40 ethanol/60 water and 60 ethanol/40 water.

By way of natural polar compounds of interest present in the aqueous extract of *Paeonia suffruticosa Yao Huang* according to the invention, particular mention may be made of:
- sugars (glucose, fructose, possibly saccharose) and vitamin C, at the core of the skin's energy processes,
- organic acids (citric acid, malic acid), polyphenols (catechin), carriers of anti-radical activities,
- minerals, combined with skin protection and repair systems, and
- amino acids, source of vitality and protein constituents for the cell.

The inventors have indeed demonstrated that the aqueous extracts of *Paeonia suffruticosa* Yao Huang contain several compounds, for example secondary metabolites which are of interest for the skin. Particularly, the aqueous extract of *Paeonia suffruticosa* Yao Huang contains compounds selected from the group of: flavonoids, phenols, tannins, monoterpenoid glycosides and paeonols. Particularly, the flavonoids are selected from quercetin, kaempferol, myricetin and isorhamnetin.

According to one embodiment the aqueous extract obtained from the flowers of *Paeonia suffruticosa* Yao Huang contains one or more flavonoid selected from quercetin, kaempferol, myricetin and isorhamnetin. Particularly, the aqueous extract obtained from the flowers of *Paeonia suffruticosa Yao Huang* contains kaempferol and myricetin.

According to another embodiment, the aqueous extract obtained from the leaves of *Paeonia suffruticosa Yao Huang* contains one or more metabolites compounds which are phenols and/or tannins.

According to another embodiment, the aqueous extract obtained from the roots of *Paeonia suffruticosa Yao Huang* contains one or more monoterpenoid glycosides and/or paeonols.

The aqueous extract of *Paeonia suffruticosa Yao Huang* according to the invention may be obtained by various processes known to the skilled person, particularly by those disclosed in Example.

### ➢ Hydroalcoholic extract

The hydroalcoholic extract of *Paeonia suffruticosa Yao Huang* according to the invention is an extract comprising aqueous solvent and another polar solvent. According to the present invention, the expression "polar solvent" means that the solvent has a polarity index value or Hildebrand total solubility parameter greater than or equal to 10. The polarity index is a quantity calculated on the basis of thermodynamic quantities (of solubility and change of state) which highlights the more or less polar nature of a molecule. By way of examples, the following solvents have a Hildebrand total solubility parameter of:
- 7.3 for hexane (apolar solvent),
- 11.6 for butylene glycol, 12.7 for ethanol and 16.5 for glycerol (polar solvents),
- 23.4 for water H₂O (polar solvent).

The preferred polar solvents are those consisting of a compound containing at least one polar covalent bond of the O-H type. As a particularly preferred polar solvent, one solvent or a mixture of solvents will be used, selected from water, C₁-C₅ alcohols, such as ethanol, glycols, such as ethylene glycol, glycerol, butylene glycol and propylene glycol, and mixtures thereof. Advantageously, water, ethanol or mixtures thereof will be used.

According to one preferred embodiment, an hydroalcoholic extract of *Paeonia suffruticosa Yao Huang* is obtained by extraction with a water (as a co-solvent) and C₁-C₅ alcohols such as methanol, ethanol, glycols, such as ethylene glycol, glycerol, butylene glycol and propylene glycol, and mixtures thereof. Particularly, for the hydroalcoholic extraction, methanol or ethanol is used, more particularly ethanol.

According to one embodiment, the hydroalcoholic extract of *Paeonia suffruticosa Yao Huang* is obtained from the whole plant, or preferably from the leaves, from the flowers, from the roots or from the seed pods, more preferably from the leaves or from the flowers and the extraction uses a cosmetically acceptable polar solvent selected from C1-C5 mono-alcohol and water, in particular a mixture of ethanol and water, more preferably in a mass ratio between 40 ethanol/60 water and 60 ethanol/40 water.

According to another embodiment, the hydroalcoholic extract of *Paeonia suffruticosa Yao Huang* comprises 5% to 20% by weight of *Paeonia suffruticosa Yao Huang* (active ingredient), 30% to 50% water, 50% to 70% ethanol, in particular 10 % *Paeonia suffruticosa Yao Huang* (active ingredient), 30 % water, 60% ethanol and preservatives qs 100%.

The inventors have indeed demonstrated that the hydroalcoholic extract of *Paeonia suffruticosa Yao Huang* contains several compounds, for example secondary metabolites which are of interest for the skin. Particularly, the hydroalcoholic extract of *Paeonia suffruticosa Yao Huang* contains compounds selected from the group of: flavonoids, phenols, tannins, monoterpenoid glycosides and paeonols. Particularly, the flavonoids are selected from quercetin, kaempferol, myricetin and isorhamnetin.

Thus, according to one embodiment, the hydroalcoholic extract of *Paeonia suffruticosa Yao Huang* of the invention contains:
- flavonoids selected in the group consisting of quercetin, kaempferol, myricetin and isorhamnetin and mixtures thereof, in particular kaempferol and isorhamnetin, and
- metabolites compounds selected in the group consisting of phenols, tannins, monoterpenoid glycoside and paeonols, and mixtures thereof.

According to particular embodiment the hydroalcoholic extract obtained from the flowers of *Paeonia suffruticosa Yao Huang* contains one or more flavonoid selected from quercetin, kaempferol, myricetin and isorhamnetin. Particularly, the a hydroalcoholic extract obtained from the flowers of *Paeonia suffruticosa Yao Huang* contains kaempferol and myricetin.

According to another particular embodiment, the hydroalcoholic extract obtained from the leaves of *Paeonia suffruticosa Yao Huang* contains one or more metabolites compounds which are phenols and/or tannins.

According to another embodiment, the hydroalcoholic extract obtained from the roots of *Paeonia suffruticosa Yao Huang* contains one or more monoterpenoid glycosides and tannins and paeonols.

The content of the above-mentioned compounds is higher in the hydroalcoholic extract of *Paeonia suffruticosa Yao Huang* compared to the aqueous extract of this plant. Thus, according to one preferred embodiment the cosmetic composition of the present invention contains a hydroalcoholic extract of *Paeonia* Yao Huang.

The hydroalcoholic extract of *Paeonia suffruticosa Yao Huang* extract can be prepared by various extraction processes known to the skilled person, involving the steps of grinding the plant material, dispersion of the ground material in a polar solvent, separation of the soluble and insoluble phases by filtration, concentration and possible resolution.

In particular, the plant material is extracted using a solvent consisting of water, with or without a pH modifier (e.g., organic acid, citric acid, hydrochloric acid), and C1-C5 mono-alcohol, particularly an ethanol in a mass ratio between 40 ethanol/60 water and 60 ethanol/40 water.

Particularly, the mass ratio between the solvents and the plant material may comprise 5% to 20% by weight of *Paeonia suffruticosa Yao Huang* (active ingredient), 30% to 50% water, 50% to 70% ethanol, in particular 10 % *Paeonia suffruticosa Yao Huang* (active ingredient), 30 % water, 60% ethanol and preservatives qs 100%.

Extraction can be carried out at high temperature by reflux or by maceration at room temperature.

Extraction can be done using well-known physicochemical techniques such as ultrasound, microwave, extrusion, pulsed electric field, and/or cryoextraction.

In the case of an ultrasound extraction, the duration of the extraction may range from 2 to 10 minutes, in particular 5 minutes.

In the case of an extraction using another technology among those mentioned above, the duration may generally range from 1h to 10h, in particular 2 to 6h, or even 4h, in particular at 60°C.

The extraction process advantageously includes a filtration step to separate the liquid phase from the depleted plant material. The skilled person will choose suitable filtration sieves, in particular with filtration diameters from 0.1 µm to 0.5 µm, in particular from 0.2 to 0.3 µm.

The cycle of extraction and filtration can be repeated several times in order to deplete the plant material of substances with an affinity for the extraction solvent.

The extraction process can be further completed by a step of partial or total removal of the extraction solvents.

The extract can be advantageously concentrated by removing a portion of the solvent or extraction solvent mixture.

It is thus possible to obtain an aqueous concentrate free of a significant amount of organic solvents or, by removing all the extraction solvent, to obtain a dry residue.

Alternatively, the product of the extraction step can be freeze-dried or atomized to form a powder.

Thus, according to a particular and preferred embodiment of the invention, the hydroalcoholic extract *Paeonia suffruticosa Yao Huang* is obtained by the extraction process comprising the following steps:
a) harvesting whole fresh plants of *Paeonia* Yao Huang, optionally stored at -20°C,
b) separating the flowers, the leaves, the roots and the seed pods;
c) freeze-drying the plant parts for 24 hours at - 45°C
d) macerating 5 to 20%, in particular 10% of plant material in a solvent comprising 30% to 50% water, 50% to 70% ethanol, particularly at temperature comprised between 40°C and 60°C,
e) extraction optionally under stirring,
f) liquid/solid separation,
g) optional addition of preservatives,
h) filtration, especially to 0.22 µm,
i) optionally packaging, under nitrogen if necessary,
j) optionally storage at +4°C.

According to a particular and preferred embodiment of the invention, the cosmetic composition according to the invention is characterized in that the hydroalcoholic extract of *Paeonia suffruticosa Yao Huang* comprises an extract of *Paeonia suffruticosa Yao Huang* in a polar solvent, in particular in a weight ratio of 10:90 (plant extract:polar solvent).

The aqueous or the hydroalcoholic extract of *Paeonia suffruticosa Yao Huang* is present in the cosmetic composition in a content ranging from 0.01% to 95%, in particular from 0.01% to 20%, preferably from 0.05% to 10% and more preferably from 0,05% to 5% by weight of raw material based on the total weight of said composition.

The dry extract of *Paeonia suffruticosa Yao Huang* is present in the aqueous or hydroalcoholic extract in a content ranging from 1% to 20%, particularly between 5% and 15% and more particularly 5% to 10%, in particular 10%, by weight of the total weight of the aqueous or hydroalcoholic extract.

So according to an embodiment, a composition according to the invention comprises a content of dry matter of *Paeonia suffruticosa Yao Huang* ranging from 0.001% to 9,5%, in particular from 0.001% to 2%, preferably from 0.005% to 1% and more preferably from 0,005% to 0,5% by weight of dry matter based on the total weight of said composition.

### Galenic

The cosmetic composition of the invention may be in any galenic form suitable for topical application to the skin, for example a serum, an oil-in-water emulsion, a water-in-oil emulsion, a multiple emulsion, or an aqueous gel.

The composition is preferentially intended to be applied to the face and is provided for example in the form of a lotion, a care cream, a serum, a facial fluid, or a care gel for the face.

The aqueous phase generally represents 1 to 99% by weight, based on the total weight of said composition.

### Aqueous phase

The aqueous phase of the composition according to the invention comprises water and optionally a water-soluble solvent.
'Water-soluble solvent' according to the invention means a compound which is liquid at room temperature and miscible with water (miscibility in water of more than 50% by weight at 25° C and atmospheric pressure). Particular mention may be made of:
- lower mono-alcohols C₁-C₅ such as ethanol, isopropanol and mixtures thereof;
- C₂-C₈ glycols such as ethylene glycol, propylene glycol, 1,3-butylene glycol, dipropylene glycol, and mixtures thereof;
- C₂-C₃₂ polyols such as polyglycerols, polyethylene glycols, and mixtures thereof, and mixtures thereof.

It may also include hydrophilic gelling agents, antioxidants, preservatives and mixtures thereof.

### Fat or oil phase

The cosmetic composition of the invention may also include a fat (solid fat) or oil phase.
"Oil phase" means an oil or a mixture of oils that are miscible with each other. "Oil" means, in the sense of the invention, a fatty substance, not soluble in water, liquid at 25° C.

An oil phase according to the invention may comprise hydrocarbon, silicone oils, and mixtures thereof. These oils can be volatile or non-volatile, vegetable, mineral or synthetic.

The oils may be present in the composition of the invention in a content ranging from 0.01 to 95% by weight based on the total weight of the composition.

The fat or oil phase may also include lipophilic gelling agents, film-forming polymers, surfactants, antioxidants and mixtures thereof.

### Additional ingredients

The composition of the invention may also include any additive commonly used in cosmetics such as antioxidants, perfumes, cosmetic active agents, such as emollients, moisturizers, vitamins, anti-aging agents, lightening agents, fillers, pearlescent agents and mixtures thereof.

### Cosmetic process

The invention also relates to a cosmetic process for caring the keratinic materials of a subject in need thereof, in particular skin and/or lips, comprising the application onto the keratinic materials, in particular skin and/or lips of a cosmetic composition as defined according to the invention comprising an aqueous or hydroalcoholic extract of *Paeonia* Yao Huang.

According to one embodiment, the cosmetic process of the invention comprises applying the cosmetic composition of the invention onto keratinic materials of a subject in need thereof, for anti-aging, to promote and/or improve the barrier function, densify the extracellular matrix and/or reduce its degradation, improve the elasticity and/or firmness of the skin, improve the radiance and/or homogeneity of the skin, improve the collagen synthesis in the skin and/or prevent and/or reduce the degradation of collagen, reduce the production of metalloproteases, and/or reduce the formation of wrinkles and/or fine lines.

The composition can be applied to the body, face and/or neck. According to a particular embodiment, the composition is applied to the face and/or neck.

Preferably, a cosmetic composition comprising a hydroalcoholic extract of *Paeonia* Yao Huang, will be used in these cosmetic processes.

### Cosmetic uses

The invention also relates to the use of at least one effective amount of at least one aqueous or hydroalcoholic extract of *Paeonia* Yao Huang, in particular an hydroalcoholic extract of *Paeonia* Yao Huang, as an agent for anti-aging, intended to promote and/or improve the barrier function, densify the extracellular matrix and/or reduce its degradation, improve the elasticity and/or firmness of the skin, improve the radiance and/or homogeneity of the skin, improve the collagen synthesis in the skin and/or prevent and/or reduce the degradation of collagen, reduce the production of metalloproteases, and/or reduce the formation of wrinkles and/or fine lines.

The aqueous or hydroalcoholic extract of *Paeonia suffruticosa Yao Huang* used according to the invention is as described above.

The invention will now be illustrated in the following non-limiting examples and the results shown in the figures.

### DESCRIPTION OF THE DRAWINGS

**Figure 1****:** The content of flavonoids of the flowers samples: **AH** is the water extract of flowers, **BH** is 60% ethanol water extract of flowers. **Qu:** quercetin, **Ka:** kaempferol, **My:** myricetin, **Is:** isorhamnetin. **YH:** *Paeonia* Yao Huang.
**Figure 2****:** The content of metabolite in the leaves samples: **AY** is the water extract of leaves, **BY** is 60% ethanol water extract of leaves; **CY** is the ethanol extract of leaves. **Ph:** phenols; **Ta:** tannins. **YH**: *Paeonia* Yao Huang.
**Figure 3****:** The content of metabolite in the roots samples: **AG** is the water extract of roots, **BG** is 60% ethanol water extract of roots; **CG** is the ethanol extract of roots. **Ph:** phenols, **Mo:** monoterpenoid glycosides, **Ta:** tannins, **Pa:** paeonols. **YH**: *Paeonia* Yao Huang.

### EXAMPLES

### Example 1: Preparation of extracts of Paeonia suffruticosa Yao Huang from the flowers, the leaves, the roots and the seed pods

### • Plant material

Flowers, leaves, seed pods and root samples from *Paeonia suffruticosa Yao Huang* have been collected from Institute of Botany, Chinese Academy of Sciences (CAS), China from April to October, 2019. All samples have been identified and certificate specimens have been deposited in the Institute of Botany. All flowers at full bloom stage have been collected. Fresh flowers have been frozen-dried for 24 hours to constant weight with FD-1T vacuum freeze-drying agent (Beijing Oriental Jinrui Science and Technology Development Co., Ltd., Beijing) at - 45°C. The leaves, seed pods and roots from three independent adult plants have been selected, separately bagged in nylon mesh bags (22 cm×15 cm, Beijing Kechuang Xinda Science and Technology Co., Ltd., Beijing, China), and dried at room temperature (about 20°C) for about one month until constant weight. Finally, they have been stored in a brown dryer containing silica gel desiccant at room temperature for storage.

### • Aqueous extract of Paeonia suffruticosa Yao Huang

The aqueous extract of *Paeonia suffruticosa Yao Huang* is obtained by extraction using water according to known methods. The aqueous extract of Paeonia suffruticosa Yao Huang is obtained comprising 10% by weight of dry matter (active ingredient), 90% by weight of water, and preservatives qs 100%.

### • Hydroalcoholic extract of Paeonia Yao Huang

The different parts (flowers, leaves, roots and seed pods) of *Paeonia suffruticosa Yao Huang* have been separated. Then, they have been separately macerated in a hydroalcoholic solution comprising 60% ethyl alcohol + 40% distilled water during 30 to 90 minutes at 40 to 60 °C. The maceration is performed in ratios 1/5 to 1/20 of plant material/hydroalcoholic solvent. At the end of the maceration period, the obtained liquid is drained, the wet grounds are pressed and then filtered. The liquid phase is separated from the solid phase.

### Example 2: Phytochemical characterization of extracts of Paeonia suffruticosa Yao Huang obtained from the flowers, leaves and roots

### 1. Materials and methods

### • Plant material

The plant material as described in Example 1 have been used for performing the phytochemical characterization of extracts of Paeonia Yao Huang.

### • Chemicals and standards

Several standards have been used, all standards' purity surpass 98%. Rutin, gallic acid and paeoniflorin have been purchased from ANPEL Laboratory Technologies Inc. (Shanghai, China). Oxypaeoniflorin, 1,2,3,4,6-penta-O-galloyol-β-D-glucose, albiflorin, benzoylpaeoniflorin, methyl gallate and paeonol have been purchased from Shanghai Tauto Biotech (Shanghai, China). Apiopaeonoside was purchased from Chengdu Pusi Biotechnology Co., Ltd. (Chengdu, China). Quercetin was purchased from the National Institute for the Control of Pharmaceutical and Biological Products (Beijing, China). Methanol, which was of chromatographic grade, was purchased from Alltech Scientific (Beijing, China). Chromatographic grade acetonitrile and formic acid have been purchased from Fisher Scientific International Inc. (Fair Lawn, NJ, USA). HPLC-grade distilled water was obtained from a Milli-Q System (Millipore Corp., Billerica, MA, USA).

### • Pretreatment

Sample have been ground into a powder with liquid nitrogen in a pestle with a mortar. Sample powders have been packed into zip-lock bags, sealed under vacuum, and stored at -40°C until use.

### • Extraction of root secondary metabolites

Approximately 0.2 g dry flowers power, extracted with 1 ml 0.2% formic acid methanol (v/v) in a test tube. Leaves, seed pods and root metabolites have been extracted separately. About 0.2 g of powder sample was accurately weighed. Each sample was placed into a separate test tube, and 1 mL of extraction solution (distilled water, 60% ethyl alcohol + 40% distilled water, v/v, ethyl alcohol, respectively) was added. The tubes have been treated in a KQ-500DE ultrasonic cleaner (Ultrasonic Instruments, Kunshan, China) at 40 KHz and 20°C for 20 min, then centrifuged in a SIGMA 3K30 centrifuge (Sigma, Berlin, Germany) at 10,000 × g for 10 min. The supernatants have been collected into new test tubes. This operation (ultrasonication and centrifugation) was repeated three times, each time adding an additional 1 ml of extraction solution to the lower sediment. A total of 6 mL of extraction solution was added. Each supernatant was filtered through a 0.22 µm reinforced nylon membrane filter (ANPEL Scientific Instruments) before HPLC analysis. Three experimental replicates have been prepared for each sample.

### • HPLC-DAD

A Waters Alliance 2695 HPLC system (Waters Corp., Milford, MA, USA) equipped with an online vacuum degasser, a quaternary pump, an autosampler, a thermostatic column compartment, a Waters 996 diode array detector and an ODS-80Ts QA C18 column (250 mm × 4.6 mm; Tosoh, Tokyo, Japan), which was connected to Empower 3 (Waters Corp.). Eluent A was formic acid: water = 2:98 (v/v) and eluent B was formic acid: acetonitrile = 0.2: 99.8 (v/v). The following gradient elution was used for flowers: 8% B at 0 min, 17% B at 25 min, 17% B at 45min, 35% B at 50 min, 47% B at 54 min, 50% B at 60 min, 60% at 62 min, 95% B at 65 min, 9% B at 66 min and 9% B at 70 min. The flow rate was 0.8 ml/min. The following gradient elution was used for leaves, seed pods and roots: 0-4 min, 5% B; 4-12 min, 5%-14% B; 12-35 min, 14% B; 35-46 min, 14-26% B; 46-60 min, 26% B; 60-85 min, 26-40% B, 85-86 min, 40-5%B; 8695 min, 5% B. The flow rate was 0.6 mL/min and 10 µL aliquots of analytes have been injected. Column temperature was maintained at 30°C for all analyses. The photodiode array spectra have been recorded from 200 to 800 nm.

### • HPLC-Q-TOF-LC/MS

An Agilent 6520 HPLC system coupled to an accurate-mass Q-TOF-LC/MS (Agilent Technologies) operating in negative electrospray ionization (ESI) mode was used for the analysis. The parameters for MS detection have been as follows: scan range was m/z 100-2000 Da, nitrogen flow rate was 12 L/min, drying gas temperature was 350°C, nebulizer pressure was 35 psi and capillary voltage was 3500 V. Data processing and analysis have been managed by using Masshunter Qualitative Analysis Software B.04.00 (Agilent Technologies). The LC conditions, mobile phase composition and elution procedure have been the same as HPLC-DAD.

### • Quantitative analysis of metabolites

The content of flavonoids and anthocyanins in dried flower was calculated by semiquantitative Rutin and Cy3G5G, respectively. Standards of Rutin and Cy3G5G have been accurately weighed, dissolved in 0.2% formic acid ethyl alcohol (v/v) and in methanol, and finally diluted to the appropriate concentrations to establish calibration curves at 350 nm. Seven concentrations (1 mg/ ml, 0.5 mg/mL, 0.25 mg/mL, 0.125 mg/mL, 0.0625 mg/mL, 0.03125 mg/mL, 0.015625 mg/mL) have been prepared. The resulting regression equations of the two standers have been Y = 3×107X+59583 (R2 = 0.9996) and Y = 3×107X (R2 = 0.9989), which showed strong linearity between concentrations and peak areas.

All samples have been analyzed by HPLC-DAD, and the contents of metabolites in the leaves, seed pods and roots have been calculated based on a linear regression of HPLC-DAD peak area of commercial standards. All standards have been used for the semiquantitative assessment of all metabolites. Standards of gallic acid, paeoniflorin, oxypaeoniflorin, albiflorin, 1,2,3,4,6-penta-O-galloyol-β-D-glucose, benzoylpaeoniflorin, quercetin and paeonol have been accurately weighed, dissolved in ethyl alcohol, and finally diluted to appropriate concentrations to establish standard calibration curves. Seven concentrations (1, 0.5, 0.25, 0.125, 0.0625, 0.03125, and 0.015625 mg·/ mL) have been prepared. Quercetin was quantified at 350 nm. Gallic acid was quantified at 280 nm, and other phenols have been quantified by using gallic acid as the standard at 280 nm. Paeoniflorin, oxypaeoniflorin, albiflorin and benzoylpaeoniflorin have been quantified at 230 nm. Other monoterpene glycosides have been quantified by using paeoniforin as the standard at 230 nm. Tannins have been quantified by using 1,2,3,4,6-penta-O-galloyol-β-D-glucose as the standard at 280 nm. Paeonol was used as the standard for the quantification of paeonols. Standards of methyl gallate, and apiopaeonoside and have been dissolved in 80% methanol (v/v), which have been used for co-elution to identify the components of the sample extract.

### • Statistical analysis

Mean values have been obtained from three replicates of each sample and used for further analysis. The samples have been clustered according to the contents of metabolites in root extracts. Variations in five types of metabolites (monoterpenoid glycosides, phenols, paeonols, tannins and flavonoids) have been determined by one-way analysis of variance, the exact statistical test was employed by using LSD, at p < 0.05. This analysis was conducted using SPSS 19.0 (SPSS Inc., Chicago, IL, USA). SigmaPlot 12.0 (SigmaPlot, Systat Software Inc., Erkrath, Germany) was used to plot graphs.

### ➢ Results

### • Flowers

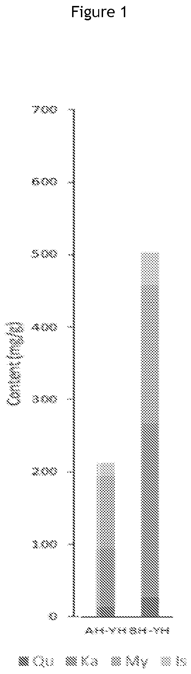

Several flavonoids have been separated at 350 nm and 520 nm (Table 3). The identification of compounds is mainly based on retention time, elution sequence, ultraviolet-visible absorption spectrum characteristics and mass spectrometry fragments information, which are compared with standard products or references. Four particular flavonols have been found: quercetin, kaempferol, myricetin and isorhamnetin (**Figure 1**).

The extraction efficiency of the three kinds of extracts have been significantly different, among which the extraction efficiency of ethanol was the lowest, followed by water, and the extraction efficiency of 60% ethanol water (V/ V) was the highest. The content of kaempferol (Ka) is 240.612 mg/g and the content of isorhamnetin (IS) is 190.59 mg/g.

### • Leaves

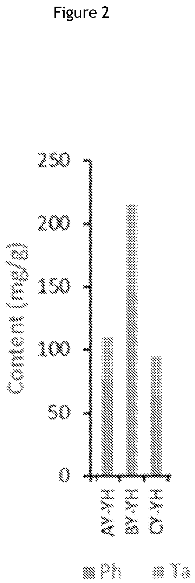

Among secondary metabolites tannins and phenols have been detected (**Figure 2**). The extraction efficiency of 60% ethanol water extract has been the highest from three kinds of samples except.

### • Root

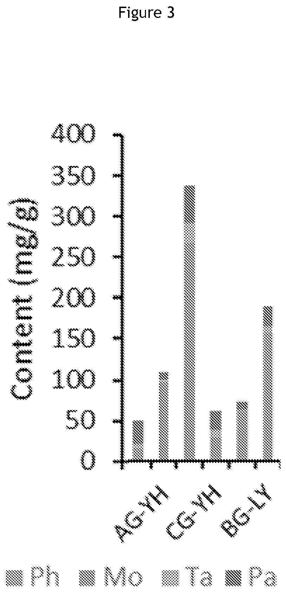

By optimizing the procedure, the metabolites in the root samples have been separated as much as possible. The secondary metabolites have been identified at 254 nm. They are: monoterpenoid glycosides, phenols, paeonols and tannins (**Figure 3**).

Compared with other samples, the extraction efficiency of 60% ethanol water extract was the highest. The total secondary metabolites content in this extract is 335.81 mg/g. In the root sample extract, the content of monoterpenoid glycosides is 266.08 mg/g.

***Example 3: The extracts of Paeonia suffruticosa Yao Huang are able to increase the synthesis of filagrine (FLG) in granular layer and stratum corneum of the skin. These extracts also decrease ROS and increase collagen I synthesis.***

The effect of the different extracts of *Paeonia suffruticosa Yao Huang* have been assessed using a reconstituted skin models (an *in vitro* model of reconstituted skin with a fully developed barrier). The effect of UV irradiation on the skin model has been assessed in four experimental groups. The used experimental groups are as follows: Blank control group (BC), Negative control group (NC), Positive control group (PC) and Sample group. 3 biological replicates in each group has been used.

Positive control group is constituted by the product WY14643 which is an activator of PPARα (a member of the PPAR family). PPAR is a nuclear transcription factor. When the ligand (activator) binds to PPAR, the two enter the nucleus as a complex and then bind to the promoter region of the downstream target gene to initiate the transcription of the gene. Target genes mainly include genes related to cell proliferation and differentiation and genes related to lipid synthesis and metabolism, while the transcription of lipids and proteins related to barrier composition is also regulated by PPAR. Therefore, PPAR activators have the function of barrier repair.

The experimental design is given in **Table 1** below:

| **Groups** | **Sample Information** | | **Detection system** | | | |
|---|---|---|---|---|---|---|
| | **Sample name** | **Concentration** | **Induction conditions** | **Test index** | **Detection model** | **Test method** |
| Blank control | BC | / | / | | | |
| Negative control | NC | / | 200mJ/cm² UVB (Continuous irradiation 3 days) | | | |
| Positive control | PC (WY14643) | 50µM | | 1.Morphometry | | 1.H&E |
| | | | | 2.FLG | EpiKutis^{®} | 2.IF |
| Sample | 1# | 1.563% | | | | 3.IF |
| | 2# | 0.781% | | | | |
| | 3# | 0.781% | | | | |
| | 4# | 0.781% | | | | |
| | 5# | 0.781% | | | | |

### ➢ Morphometric results

Chronic UV exposure can affect the differentiation of keratinocytes, resulting in abnormal differentiation, hyperkeratinization (thickening of the stratum corneum), and accompanied by Parakeratosis, that is, cells in the stratum corneum contain concentrated nuclei that have not disappeared.

Compared with the NC group (UVB irradiation group), hyperkeratinization (thickening of the stratum corneum) and Parakeratosis (cells in the stratum corneum contain concentrated nuclei that have not disappeared) in the PC (WY14643) group the morphological aspect of the stratum corneum have been improved.

Compared with the NC group (UVB irradiation group), hyperkeratinization (thickening of the stratum corneum) and Parakeratosis (cells in the stratum corneum contain concentrated nuclei that have not disappeared) the extracts of *Paeonia suffruticosa Yao Huang* allow to reestablish the normal morphological aspect of stratum corneum. These results have been confirmed by the results obtained when measuring FLG (separate stratum and stratum corneum) optical densities.

### ➢ FLG (filaggrin) protein results

**Table 2: Granular layer FLG integral optical density values (IOD)summary**

| Sample name | Relative IOD average | SD | P value(*vs* BC) | P value(*vs* NC) |
|---|---|---|---|---|
| BC | 1.00 | 0.04 | / | / |
| NC | 0.25 | 0.09 | < 0.0001#### | / |
| PC | 0.67 | 0.02 | / | 0.0342* |
| 1# | 0.51 | 0.16 | / | 0.4317 |
| 2# | 0.86 | 0.41 | / | 0.0005*** |
| 3# | 0.38 | 0.07 | / | 0.9869 |
| 4# | 0.19 | 0.09 | / | 0.9994 |
| 5# | 0.10 | 0.04 | / | 0.9661 |

| | | | | |
|---|---|---|---|---|
| Note: **BC:** blank control; **NC:** negative control; **PC:** positive control (WY14643); **1#:** extract from the flowers of Paeonia Yao Huang; **2#:** extract from the leaves of Paeonia Yao Huang; **3#:** extract from the roots of Paeonia Yao Huang; **4#:** Paeonia Luo Yangf Hong and **5#:** Paeonia Feng Dan. Use one-way ANVOA methods for statistical analysis, NC group compared with the BC group, significance with #, p-value<0.0001 is expressed as ####;PC group and sample group compared to the NC group, significance with *, p-value<0.01 is expressed as**,P-value< 0.0001 is expressed as**** | | | | |

As shown in **Table 2** above, compared with the blank control (BC group), the content of FLG protein decreased significantly after UVB irradiation (NC group). After PC (WY14643) treatment, FLG protein content significantly increased. The same results are observed with samples containing Paeonia suffruticosa Yao Huang obtained from the flowers, the leaves and the roots (samples 1#, 2# and 3#). This effect is significative with leaves sample (sample 2#).

The increasing effect on FLG protein obtained with the samples of Paeonia suffruticosa Yao Huang has been compared to the effect obtained from other extracts obtained from different species of Paeonia (called Luo Yangf Hong and Feng Dan and corresponding to samples 4# and 5# respectively). It may be observed in **Table 2** that, the levees extract of Paeonia suffruticosa Yao Huang allow to significantly improve the concentration of FLG protein compared to the extracts obtained from others species of Paeonia.

**Table 3: Granular layer and the stratum corneum FLG integral optical density values (IOD) summary**

| Sample name | Relative IOD average | SD | P value(*vs* BC) | P value(*vs* NC) |
|---|---|---|---|---|
| BC | 1.00 | 0.28 | / | / |
| NC | 0.32 | 0.02 | 0.0012## | / |
| PC | 0.80 | 0.02 | / | 0.0475* |
| 1# | 0.99 | 0.08 | / | 0.0015** |
| 2# | 1.09 | 0.39 | / | 0.0002*** |
| 3# | 0.77 | 0.36 | / | 0.0690 |
| 4# | 0.76 | 0.04 | / | 0.0892 |
| 5# | 0.27 | 0.03 | / | 0.9996 |

| | | | | |
|---|---|---|---|---|
| Note: **BC:** blank control; **NC:** negative control; **PC:** positive control (WY14643); **1#:** extract from the flowers of Paeonia Yao Huang; **2#:** extract from the leaves of Paeonia Yao Huang; 3#: extract from the roots of Paeonia Yao Huang; **4#:** Paeonia Luo Yangf Hong and **5#:** Paeonia Feng Dan. Use one-way ANVOA methods for statistical analysis, NC group compared with the BC group, significance with #, p-value<0.01 is expressed as ##;PC group and sample group compared to the NC group, significance with *,p-value<0.05 is expressed as*, p-value<0.01 is expressed as**,P-value<0.001 is expressed as***,P-value<0.0001 is expressed as**** | | | | |

**Table 3** shows that compared with the blank control (BC group), the content of FLG protein decreased significantly after UVB irradiation (NC group). After PC (WY14643) treatment, FLG protein content significantly increased. Similar increase of the concentration is also observed with samples containing *Paeonia suffruticosa* Yao Huang obtained from the flowers, the leaves and the roots (samples 1#, 2# and 3#).

The increasing effect on FLG protein in stratum corneum obtained with the samples of *Paeonia suffruticosa* Yao Huang has been compared to the effect obtained from other extracts obtained from different species of Paeonia (called Luo Yangf Hong and Feng Dan and corresponding to samples 4# and 5# respectively). It may be observed on **Table 3** that, the extract of Paeonia suffruticosa Yao Huang allow to significantly improve the concentration of FLG protein in stratum corneum compared to the extracts obtained from others species of Paeonia.

### ➢ Effect of the extract of Paeonia suffruticosa Yao Huang on ROS and collagen I on the photoaging of the skin

One of the most significant mechanisms of photoaging is the degradation of collagen induced by UV irradiation, that is, the activation of the AP-1 signaling pathway by UV irradiation can induce the production of matrix metalloproteinases, which can degrade collagen, thereby reducing the collagen content in the skin, leading to wrinkles and other aging symptoms. For assessing the effect of *Paeonia suffruticosa* Yao Huang extract, fibroblasts have been used as the research model, and an in vitro photoaging model was established by UVA irradiation. ROS (Reactive Oxygen Species) and collagen 1 contents have been detected to determine the anti-photoaging effect of the compounds to be tested.

**Table 4: Experimental Design**

| **Groups** | **Sample Information** | | **Detection system** | | | |
|---|---|---|---|---|---|---|
| | **Sample name** | **Concentration** | **Induction conditions** | **Test index** | **Detection model** | **Test method** |
| Blank control | BC | / | / | 1.ROS | Fibroblasts | 1.FCM |
| Negative control | NC | / | 30mJ/cm² UVA | | | |
| Positive control 1 | PC (VE-0.05%) | 0.05% | | 4.Collagen | | 2.ELISA |

| Positive control 2 | PC (TGFβ-50ng/mL) | 50ng/mL | | | | |
|---|---|---|---|---|---|---|
| Sample | 1# | 1.25% | | | | |
| | 2# | 1.25% | | | | |
| | 3# | 1.156% | | | | |
| | 4# | 1.25% | | | | |

### • ROS

**Table 5: ROS data collection**

| Sample name | ROS(MFI) content average | SD | P value(*vs* BC) | P value(*vs* NC) |
|---|---|---|---|---|
| BC | 109450.13 | 3580.03 | / | / |
| N C | 456852.90 | 7779.09 | ## < 0.0001 ## | / |
| PC(VE-0.05%) | 248401.00 | 31741.18 | / | < 0.0001**** |
| 1# | 361385.07 | 19593.17 | / | 0.0448* |
| 2# | 245340.70 | 60088.90 | / | <0.0001*** * |
| 3# | 533150.83 | 27500.36 | / | 0.1821 |
| 4# | 465654.13 | 81268.76 | / | 0.9996 |

| | | | | |
|---|---|---|---|---|
| Note : **BC:** blank control; **NC:** negative control; **PC:** positive control (VE-0.05%); **1#** : extract from the flowers of Paeonia Yao Huang; **2#:** extract from the leaves of Paeonia Yao Huang; **3#:** extract from the roots of Paeonia Yao Huang; **4#:** Paeonia HEI Tian E. Use one-way ANVOA methods for statistical analysis, NC group compared with the BC group, significance with #, p-value<0.0001 is expressed as ####;PC group and sample group compared to the NC group, significance with *, p-value<0.01 is expressed as**,P-value 0.0001 is expressed as**** | | | | |

**Table 5** shows compared with the BC group, a marked increase in the ROS(MFI) content of the NC group. This demonstrates that the experimental stimulation effective. Such a significant decrease is also observed with samples containing Paeonia suffruticosa Yao Huang obtained from the flowers, the leaves and the roots (samples 1#, 2# and 3#).

The decreasing effect on ROS obtained with the samples of Paeonia suffruticosa Yao Huang has been compared to the effect obtained from another extract obtained from a different species of Paeonia (called Paeonia HEI Tian E corresponding to samples # 4). It may be observed in **Table 5** that, the extract of Paeonia suffruticosa Yao Huang allow to significantly decrease ROS production compared to the extract obtained from another specie of Paeonia.

### • Collagen I

**Table 6: Collagen I data collection**

| Sample name | Average concentration(pg/ mL) | SD | P value(*vs* BC) | P value (*vs* NC) |
|---|---|---|---|---|
| BC | 806.979 | 10.48 | / | / |
| NC | 398.921 | 28.98 | < 0.0001#### | / |
| PC(TGFB-50ng/mL) | 793.784 | 10.91 | / | < 0.0001**** |
| 1# | 554.356 | 39.62 | / | 0.0237* |
| 2# | 358.967 | 78.42 | / | 0.9902 |
| 3# | 351.846 | 22.81 | / | 0.9750 |

| | | | | |
|---|---|---|---|---|
| Note : Note : **BC:** blank control; **NC:** negative control; **PC:** positive control (TGFB-50ng/mL); **1#** : extract from the flowers of Paeonia Yao Huang; **2#:** extract from the roots of Paeonia Yao Huang; **3#:** Paeonia HEI Tian E. Use one-way ANVOA methods for statistical analysis, NC group compared with the BC group, significance with #, p-value<0.0001 is expressed as ####;PC group and sample group compared to the NC group, significance with *, p-value<0.01 is expressed as**,P-value 0.0001 is expressed as**** | | | | |

**Table 6** shows a marked decline in the Collagen I concentration of the NC group compared with the BC group. This demonstrates that the experimental stimulation effective. Compared to the NC group, with the sample of PC group, Collagen I concentration increased significantly. Such a significative increase is significative with the extract obtained from the flower of Paeonia Yao Huang.

The increasing effect on collagen I synthesis obtained with the samples of *Paeonia suffruticosa* Yao Huang has been compared to the effect obtained from another extract obtained from a different species of *Paeonia* (called Paeonia HEI Tian E corresponding to samples 3#). It may be observed in **Table 6** that, the flower extract of Paeonia suffruticosa Yao Huang allow to significantly increase collagen I production compared to the extract obtained from another specie of *Paeonia.*

### Example 4: Cosmetic formulations

### 4.1 Composition in the form of an emulsion

### Aqueous phase:

| | |
|---|---|
| Demineralized water | qs 100.0% |
| Glycols | 20.0% |
| Preservatives | 0.6% |
| Chelator | 0.04% |
| Carbomer (Carbopol^{®} 981) | 0.3% |
| Sodium polyacrylate (Covacryl^{®} MV60) | 0.2% |
| Sodium Hydroxide | 0.15% |

**Hydroalcoholic extract of *Paeonia suffruticosa* Yao Huang according to the invention* 5.0%**

### Fatty phase:

| | |
|---|---|
| Vegetable oil, esters, silicones | 16% |
| Antioxidant | 0.2% |
| Fragrance concentrate | 0.4% |
| Steareth-2 | 0.8% |
| Steareth-21 | 1.5% |

| | |
|---|---|
| * as described in the Materials and Methods section | |

The composition is prepared according to the following procedure:
- the gelling agents are dispersed in the aqueous phase which is heated to 70°C;
- the fat phase (excluding fragrance concentrate, antioxidant) is heated to 70° C;
- the emulsion is made by introducing the fat phase into the aqueous phase under strong stirring;
- the gelling agents are neutralized by adding sodium hydroxide and the emulsion is cooled under moderate stirring with the introduction of the fragrance concentrate, the antioxidant and the hydroalcoholic extract of *Paeonia suffruticosa* Yao Huang at low temperature.

Applied to the skin, especially the face, this composition brings nutrition, firmness and hydration to the skin to improve anti-age effect.

### 4.3: Composition in the form of an aqueous gel for the face

| **Hydroalcoholic extract *Paeonia suffruticosa* Yao Huang *** | **20.00%** |
|---|---|
| Preservatives | 0.50% |
| Sugar | 0.20% |
| Carbomer | 0.70% |
| Purified water | Qs 100% |
| EDTA tetrasodium powder | 0.20% |
| Sodium hydroxide | 0.17% |

| | |
|---|---|
| * as described in the Materials and Methods section | |

The hydroalcoholic extract *Paeonia suffruticosa* Yao Huang according to the invention and the preservatives are mixed and homogenized at room temperature under stirring. The sugars are added under stirring, then the carbomer, then the water and the EDTA under stirring. The aqueous gel is then neutralized with the addition of sodium hydroxide under stirring until a homogeneous gel is obtained.

Applied to the facial skin, this aqueous gel gives a fresh effect and brings suppleness and firmness to the skin and improve anti-aging effect.

## Claims

1. A cosmetic composition for topical application to the skin comprising, in a physiologically acceptable medium, at least one effective amount of at least one aqueous or hydroalcoholic extract of ***Paeonia suffruticosa*** Yao Huang, preferably an hydroalcoholic extract of ***Paeonia*** Yao Huang.

2. A cosmetic composition as claimed in claim 1, wherein the aqueous or hydroalcoholic extract of ***Paeonia suffruticosa*** Yao Huang is an extract obtained from the whole plant, or preferably, from the leaves, from the flowers, from the roots or from the seed pods, more preferably from the leaves or from the flowers.

3. The cosmetic composition as claimed in claim 1 or claim 2, wherein the aqueous or the hydroalcoholic extract of ***Paeonia suffruticosa*** Yao Huang is present in the composition in a content ranging from 0.01% to 95%, in particular from 0.01% to 20%, preferably from 0.05% to 10% and more preferably from 0,05% to 5% by weight of raw material based on the total weight of said composition.

4. The cosmetic composition as claimed in any one of claims 1 to 3, wherein the cosmetic composition is in the form of a solution, an emulsion, a serum or a gel.

5. A non-therapeutic cosmetic process for caring the keratinic materials of a subject in need thereof, in particular skin and/or lips, comprising the application to the keratinic materials, in particular skin and/or lips of a cosmetic composition as defined in any one of claims 1 to 4.

6. A cosmetic process as claimed in claim 5, wherein the cosmetic composition is applied onto keratinic materials of a subject in need thereof, for anti-aging, to promote and/or improve the barrier function, densify the extracellular matrix and/or reduce its degradation, improve the elasticity and/or firmness of the skin, improve the radiance and/or homogeneity of the skin, improve the collagen synthesis in the skin and/or prevent and/or reduce the degradation of collagen, reduce the production of metalloproteases, and/or reduce the formation of wrinkles and/or fine lines.

7. The cosmetic process according to claim 5, wherein the cosmetic composition is applied onto keratinic materials of a subject in need thereof to promote and/or improve the barrier function, densify the extracellular matrix and/or reduce its degradation, improve the elasticity and/or firmness of the skin, improve the collagen synthesis in the skin and/or prevent and/or reduce the degradation of collagen, and/or reduce the production of metalloproteases.

8. Non-therapeutic cosmetic use of at least one effective amount of at least one aqueous or hydroalcoholic extract of ***Paeonia suffruticosa*** Yao Huang, in particular an hydroalcoholic extract of ***Paeonia suffruticosa*** Yao Huang, as an agent for anti-aging, intended to promote and/or improve the barrier function, densify the extracellular matrix and/or reduce its degradation, improve the elasticity and/or firmness of the skin, improve the radiance and/or homogeneity of the skin, improve the collagen synthesis in the skin and/or prevent and/or reduce the degradation of collagen, reduce the production of metalloproteases, and/or reduce the formation of wrinkles and/or fine lines.

9. The non-therapeutic cosmetic use according to claim 8, to promote and/or improve the barrier function, densify the extracellular matrix and/or reduce its degradation, improve the elasticity and/or firmness of the skin, improve the collagen synthesis in the skin and/or prevent and/or reduce the degradation of collagen, and/or reduce the production of metalloproteases.

10. An aqueous or hydroalcoholic extract of ***Paeonia suffruticosa*** Yao Huang obtained from the whole plant, or preferably from the leaves, from the flowers, from the roots or from the seed pods, more preferably from the leaves or from the flowers and using a cosmetically acceptable polar solvent selected from C1-C5 mono-alcohol and water, in particular a mixture of ethanol and water, more preferably in a mass ratio between 40 ethanol/60 water and 60 ethanol/40 water, and
wherein said extract comprises 5% to 20% by weight of ***Paeonia suffruticosa Yao Huang*** (active ingredient), 30% to 50% water, 50% to 70% ethanol, in particular 10 % ***Paeonia suffruticosa Yao Huang*** (active ingredient), 30 % water, 60% ethanol and preservatives qs 100%.

11. The hydroalcoholic extract of ***Paeonia suffruticosa*** Yao Huang according to claim 10, containing:
- flavonoids selected in the group consisting of quercetin, kaempferol, myricetin and isorhamnetin and mixtures thereof, in particular kaempferol and isorhamnetin, and
- metabolites compounds selected in the group consisting of phenols, tannins, monoterpenoid glycoside and paeonols, and mixtures thereof.

12. The hydroalcoholic extract of ***Paeonia suffruticosa*** Yao Huang according to claim 10 or 11, obtained according to the extraction process comprising the following steps:
a) harvesting whole fresh plants of ***Paeonia suffruticosa*** Yao Huang, optionally stored at - 20°C,
b) Separating the flowers, the leaves, the roots and the seed pods;
c) freeze-drying the plant parts for 24 hours at - 45°C
d) macerating 5 to 20%, in particular 10% of plant material in a solvent comprising 30% to 50% water, 50% to 70% ethanol, particularly at temperature comprised between 40°C and 60°C,
e) extraction optionally under stirring,
f) liquid/solid separation,
g) optional addition of preservatives,
h) filtration, especially to 0.22 µm,
i) optionally packaging, under nitrogen if necessary,
j) optionally storage at +4°C.

## Patentansprüche

1. Kosmetische Zusammensetzung zum topischen Auftragen auf die Haut, umfassend, in einem physiologisch akzeptablen Medium, mindestens eine wirksame Menge mindestens eines wässrigen oder hydroalkoholischen Extrakts von Paeonia suffruticosa Yao Huang, vorzugsweise eines hydroalkoholischen Extrakts von Paeonia Yao Huang.

2. Kosmetische Zusammensetzung nach Anspruch 1, wobei das wässrige oder hydroalkoholische Extrakt von *Paeonia suffruticosa* Yao Huang ein Extrakt ist, das aus der gesamten Pflanze oder vorzugsweise aus den Blättern, aus den Blüten, aus den Wurzeln oder aus den Samenkapseln, bevorzugter aus den Blättern oder aus den Blüten, erhalten wird.

3. Kosmetische Zusammensetzung nach Anspruch 1 oder Anspruch 2, wobei das wässrige oder das hydroalkoholische Extrakt von *Paeonia suffruticosa* Yao Huang in der Zusammensetzung mit einem Gehalt von zwischen 0,01 Gew.-% und 95 Gew.-%, insbesondere zwischen 0,01 Gew.-% und 20 Gew.-%, vorzugsweise zwischen 0,05 Gew.-% und 10 Gew.-% und bevorzugter zwischen 0,05 Gew.-% und 5 Gew.-% des Rohmaterials, bezogen auf das Gesamtgewicht der Zusammensetzung, vorhanden ist.

4. Kosmetische Zusammensetzung nach einem der Ansprüche 1 bis 3, wobei die kosmetische Zusammensetzung in der Form einer Lösung, einer Emulsion, eines Serums oder eines Gels vorliegt.

5. Nicht-therapeutisches kosmetisches Verfahren zur Pflege der keratinischen Materialien, insbesondere der Haut und/oder der Lippen, einer Person, die diese benötigt, umfassend das Auftragen einer kosmetischen Zusammensetzung nach einem der Ansprüche 1 bis 4 auf die keratinischen Materialien, insbesondere die Haut und/oder Lippen.

6. Kosmetisches Verfahren nach Anspruch 5, wobei die kosmetische Zusammensetzung auf keratinische Materialien einer Person, die diese benötigt, zum Anti-Aging, zur Förderung und/oder Verbesserung der Barrierefunktion, Verdichtung der extrazellulären Matrix und/oder Verminderung der Verschlechterung ihres Zustands, Verbesserung der Elastizität und/oder Festigkeit der Haut, Verbesserung des Glanzes und/oder der Gleichmäßigkeit der Haut, Verbesserung der Kollagensynthese in der Haut und/oder Prävention und/oder Verminderung der Verschlechterung des Zustands von Kollagen, Verminderung der Produktion von Metalloproteasen und/oder Verminderung der Bildung von Falten und/oder feinen Linien aufgetragen wird.

7. Kosmetisches Verfahren nach Anspruch 5, wobei die kosmetische Zusammensetzung auf keratinische Materialien einer Person, die dies benötigt, zur Förderung und/oder Verbesserung der Barrierefunktion, Verdichtung der extrazellulären Matrix und/oder Verminderung der Verschlechterung ihres Zustands, Verbesserung der Elastizität und/oder Festigkeit der Haut, Verbesserung der Kollagensynthese in der Haut und/oder Prävention und/oder Verminderung der Verschlechterung des Zustands von Kollagen und/oder Verminderung der Produktion von Metalloprotheasen aufgetragen wird.

8. Nicht-therapeutische kosmetische Verwendung mindestens einer wirksamen Menge mindestens eines wässrigen oder hydroalkoholischen Extrakts von *Paeonia suffruticosa* Yao Huang, insbesondere eines hydroalkoholischen Extrakts von *Paeonia suffruticosa* Yao Huang, als ein Mittel zum Anti-Aging, das zur Förderung und/oder Verbesserung der Barrierefunktion, Verdichtung der extrazellulären Matrix und/oder Verminderung der Verschlechterung ihres Zustands, Verbesserung der Elastizität und/oder Festigkeit der Haut, Verbesserung des Glanzes und/oder der Gleichmäßigkeit der Haut, Verbesserung der Kollagensynthese in der Haut und/oder Prävention und/oder Verminderung der Verschlechterung des Zustands von Kollagen, Verminderung der Produktion von Metalloprotheasen und/oder Verminderung der Bildung von Falten und/oder feinen Linien bestimmt ist.

9. Nicht-therapeutische kosmetische Verwendung nach Anspruch 8 zur Förderung und/oder Verbesserung der Barrierefunktion, Dichte der extrazellulären Matrix und/oder Verminderung der Verschlechterung ihres Zustands, Verbesserung der Elastizität und/oder Festigkeit der Haut, Verbesserung der Kollagensynthese in der Haut und/oder Prävention und/oder Verminderung der Verschlechterung des Zustands von Kollagen und/oder Verminderung der Produktion von Metalloprotheasen.

10. Wässriges oder hydroalkoholisches Extrakt von *Paeonia suffruticosa* Yao Huang, das aus der gesamten Pflanze oder vorzugsweise aus den Blättern, aus den Blüten, aus den Wurzeln oder aus den Samenkapseln, bevorzugter aus den Blättern oder aus den Blüten, und unter Verwendung eines kosmetisch akzeptablen polaren Lösungsmittels, das aus C1-C5-Monoalkohol und Wasser, insbesondere einem Gemisch aus Ethanol und Wasser, ausgewählt ist, bevorzugter in einem Massenverhältnis von zwischen 40 Ethanol:60 Wasser und zwischen 60 Ethanol:40 Wasser, erhalten wird, und
wobei das Extrakt zwischen 5 Gew.-% und 20 Gew.-% *Paeonia suffruticosa* Yao Huang (Wirkstoff), zwischen 30 Gew.-% und 50 Gew.-% Wasser, zwischen 50 Gew.-% und 70 Gew.-% Ethanol, insbesondere 10 Gew.-% *Paeonia suffruticosa* Yao Huang (Wirkstoff), 30 Gew.-% Wasser, 60 Gew.-% Ethanol und Konservierungsmittel, auf 100 Gew.-% ergänzt, umfasst.

11. Hydroalkoholisches Extrakt aus *Peaonia suffruticosa* Yao Huang nach Anspruch 10, enthaltend:
- Flavonoide, die ausgewählt sind aus der Gruppe, die besteht aus Quercetin, Kaempferol, Myricetin und Isorhamnetin und Gemischen daraus, insbesondere Kaempferol und Isorhamnetin, und
- Metabolitenverbindungen, die ausgewählt sind aus der Gruppe, die besteht aus Phenolen, Tanninen, Monoterpenoidglykosid und Paeonolen und Gemischen daraus.

12. Hydroalkoholisches Extrakt aus *Paeonia suffruticosa* Yao Huang nach Anspruch 10 oder 11, das gemäß dem Extraktionsverfahren erhalten wird, das die folgenden Schritte umfasst:
a) Ernten ganzer frischer Pflanzen von *Paeonia suffruticosa* Yao Huang, die wahlweise bei -20 °C gelagert werden,
b) Trennen der Blüten, der Blätter, der Wurzeln und der Samenkapseln;
c) Gefriertrocknen der Pflanzenteile während 24 Stunden bei -45 °C
d) Mazerieren von 5 bis 20 %, insbesondere 10 %, Pflanzenmaterial in einem Lösungsmittel, das 30 % bis 50 Wasser, 50 % bis 70 % Ethanol enthält, insbesondere bei einer Temperatur von zwischen 40 °C und 60 °C,
e) Extrahieren, wahlweise unter Rühren,
f) Trennen von Flüssigkeit/Feststoffen,
g) wahlweise Beimengen von Konservierungsmitteln,
h) Filtrieren, insbesondere auf 0,22 µm,
i) wahlweise Verpacken, unter Stickstoff, falls erforderlich,
j) wahlweise Lagern bei +4 °C.

## Revendications

1. Composition cosmétique destinée à être appliquée par voie topique sur la peau, comprenant, dans un milieu physiologiquement acceptable, au moins une quantité efficace d'au moins un extrait aqueux ou hydroalcoolique de *Paeonia suffruticosa* Yao Huang, de préférence un extrait hydroalcoolique de *Paeonia* Yao Huang.

2. Composition cosmétique selon la revendication 1, dans laquelle l'extrait aqueux ou hydroalcoolique de *Paeonia suffruticosa* Yao Huang est un extrait obtenu à partir de la plante entière, ou de préférence à partir des feuilles, à partir des fleurs, à partir des racines ou à partir des follicules, mieux encore à partir des feuilles ou à partir des fleurs.

3. Composition cosmétique selon la revendication 1 ou la revendication 2, dans laquelle l'extrait aqueux ou hydroalcoolique de *Paeonia suffruticosa* Yao Huang est présent dans la composition à raison de 0,01 % à 95 %, en particulier de 0,01 % à 20 %, de préférence de 0,05 % à 10 % et mieux encore de 0,05 à 5 % en poids de matières premières par rapport au poids total de ladite composition.

4. Composition cosmétique selon l'une quelconque des revendications 1 à 3, laquelle composition cosmétique est sous la forme d'une solution, d'une émulsion, d'un sérum ou d'un gel.

5. Procédé cosmétique non thérapeutique pour prendre soin des matières kératiniques chez un sujet en ayant besoin, en particulier de la peau et/ou des lèvres, comprenant l'application aux matières kératiniques, en particulier à la peau et/ou aux lèvres, d'une composition cosmétique telle que définie dans l'une quelconque des revendications 1 à 4.

6. Procédé cosmétique selon la revendication 5, dans lequel la composition cosmétique est appliquée sur des matières kératiniques d'un sujet en ayant besoin, à des fins d'antivieillissement, pour favoriser et/ou améliorer la fonction de barrière, pour densifier la matrice extracellulaire et/ou réduire sa dégradation, pour améliorer l'élasticité et/ou la fermeté de la peau, pour améliorer l'éclat et/ou l'homogénéité de la peau, pour améliorer la synthèse du collagène dans la peau et/ou empêcher et/ou réduire la dégradation du collagène, pour réduire la production de métalloprotéases, et/ou pour réduire la formation de rides et/ou de ridules.

7. Procédé cosmétique selon la revendication 5, dans lequel la composition cosmétique est appliquée sur des matières kératiniques d'un sujet en ayant besoin, pour favoriser et/ou améliorer la fonction de barrière, pour densifier la matrice extracellulaire et/ou réduire sa dégradation, pour améliorer l'élasticité et/ou la fermeté de la peau, pour améliorer la synthèse du collagène dans la peau et/ou empêcher et/ou réduire la dégradation du collagène, et/ou pour réduire la production de métalloprotéases.

8. Usage cosmétique non thérapeutique d'au moins une quantité efficace d'au moins un extrait aqueux ou hydroalcoolique de *Paeonia suffruticosa* Yao Huang, en particulier d'un extrait hydroalcoolique de *Paeonia suffruticosa* Yao Huang, en tant qu'agent à des fins d'antivieillissement, pour favoriser et/ou améliorer la fonction de barrière, pour densifier la matrice extracellulaire et/ou réduire sa dégradation, pour améliorer l'élasticité et/ou la fermeté de la peau, pour améliorer l'éclat et/ou l'homogénéité de la peau, pour améliorer la synthèse du collagène dans la peau et/ou empêcher et/ou réduire la dégradation du collagène, pour réduire la production de métalloprotéases, et/ou pour réduire la formation de rides et/ou de ridules.

9. Usage cosmétique non thérapeutique selon la revendication 8, pour favoriser et/ou améliorer la fonction de barrière, pour densifier la matrice extracellulaire et/ou réduire sa dégradation, pour améliorer l'élasticité et/ou la fermeté de la peau, pour améliorer la synthèse du collagène dans la peau et/ou empêcher et/ou réduire la dégradation du collagène, et/ou pour réduire la production de métalloprotéases.

10. Extrait aqueux ou hydroalcoolique de *Paeonia suffruticosa* Yao Huang obtenu à partir de la plante entière, ou de préférence à partir des feuilles, à partir des fleurs, à partir des racines ou à partir des follicules, mieux encore à partir des feuilles ou à partir des fleurs, et utilisant un solvant polaire acceptable en cosmétique choisi parmi les monoalcools en C1 à C5 et l'eau, en particulier un mélange d'éthanol et d'eau, mieux encore en un rapport en masse compris entre 40 éthanol / 60 eau et 60 éthanol / 40 eau, et
lequel extrait comprend 5 % à 20 % en poids de *Paeonia suffruticosa* Yao Huang (principe actif), 30 % à 50 % d'eau, 50 % à 70 % d'éthanol, en particulier 10 % de *Paeonia suffruticosa* Yao Huang (principe actif), 30 % d'eau, 60 % d'éthanol et des conservateurs q.s. pour 100 %.

11. Extrait hydroalcoolique de *Paeonia suffruticosa* Yao Huang selon la revendication 10, contenant :
- des flavonoïdes choisis dans le groupe constitué par la quercétine, le kaempférol, la myricétine et l'isorhamnétine et leurs mélanges, en particulier le kaempférol et l'isorhamnétine, et
- des composés métabolites choisis dans le groupe constitué par les phénols, les tanins, les glycosides monoterpénoïdes et les paéonols, et leurs mélanges.

12. Extrait hydroalcoolique de *Paeonia suffruticosa* Yao Huang selon la revendication 10 ou 11, obtenu conformément au procédé d'extraction comprenant les étapes suivantes :
a) récolte de plantes fraîches entières de *Paeonia suffruticosa* Yao Huang, éventuellement stockées à -20°C,
b) séparation des fleurs, des feuilles, des racines et des follicules,
c) lyophilisation des parties de plantes pendant 24 heures à -45°C,
d) macération de 5 à 20 %, en particulier 10 % de matériau végétal dans un solvant comprenant 30 % à 50 % d'eau, 50 % à 70 % d'éthanol, en particulier à une température comprise entre 40°C et 60°C,
e) extraction éventuellement sous agitation,
f) séparation des liquides/solides,
g) éventuellement addition de conservateurs,
h) filtration, en particulier à 0,22 µm,
i) éventuellement conditionnement, sous azote si nécessaire,
j) éventuellement stockage à +4°C.
